# NEW EUROPEAN PATENT SPECIFICATION

(11) **EP 1 402 849 B2**
(45) Date of publication and mention of the opposition decision: **16.03.2011**
(45) Mention of the grant of the patent: 23.04.2008
(21) Application number: 02021067.0
(22) Date of filing: 20.09.2002
(51) Int. Cl.: A61F 2/90, A61L 31/14, A61L 31/16, B24C 1/06

(54) **Stent with rough surface and its manufacturing method**
Mit einer rauhen Oberfläche versehener Stent und sein Herstellungsverfahren
Stent à surface rugueuse et sa méthode de fabrication

(43) Date of publication of application: 31.03.2004
(73) Proprietor: Abbott Laboratories Vascular Enterprises Limited, Terrace, Dublin 2 (IE); Schömig, Albert, 80638 München (DE); Kastrati, Adnan, 81925 München (DE)
(72) Inventor: Schömig, Albert Prof. Dr., 80638 München (DE); Kastrati, Adnan Prof. Dr., 81925 München (DE); von Oepen, Randolf Dr., 72074 Tübingen (DE)
(74) Representative: Peters, Hajo

(56) References cited:
- EP-A- 0 850 604
- EP-A- 1 254 673
- EP-A2- 0 875 217
- WO-A-99/07308
- WO-A-02/083039
- DE-A- 19 916 086
- DE-A1- 10 107 339
- DE-U1- 20 200 220
- US-A- 5 891 507
- US-A- 5 972 027
- US-A- 6 099 561
- US-A1- 2002 098 278
- US-B1- 6 254 631
- US-B1- 6 387 123
- US-B1- 6 431 958

## Description

The present invention relates generally to stents which are implantable or deployable in a vascular or endoluminal location within the body of a patient to maintain the lumen open and unoccluded at that location, and in particular to improvements in stents.

First of all, stents are widely used for numerous applications where the stent is placed in the lumen of a patient and expanded. Said stents may be used in coronary or other vasculature as well as within the urinary tract, the bial tract and the intestinal tract among other body passageways and conduits.

Commonly, stents are cylindrical members which are expanded from reduced diameters to enlarged diameters. Frequently, such stents are placed on a balloon catheter with the stent in a reduced diameter state. To prevent that the balloon is damaged because of sharp corners and burrs on the surface of the stent and further to avoid thrombus formation, stents are highly polished. This is done for example by sandblasting the surface to remove said imperfections and polishing the stent afterwards to get a smooth surface. Unfortunately, the balloon catheder mounted stent can be dislodged from the uninflated balloon as a result of the navigation through the vessel of the body to the preselected site for deployment because of the highly polished surface. Furthermore, the polished and smooth surface used to avoid thrombus formation has the disadvantage, that endothelial cells have difficulties to ingrow the stent which can result in that restenosis occurs.

In the prior art, some stents are sandblasted on their interior surface to improve stent retention on the balloon.

In US-A-6 254 631 B1 a stent is disclosed, wherein the exterior surface of the stent is polished such that a smooth surface finish is achieved. The interior surface having a rough surface finish is rougher than the surface finish of the exterior surface to enhance the friction between the stent and the balloon.

Further in US-A-6 217 607 B1 a stent is disclosed which is provided with a first layer of nobel metal. Further a second outermost layer is provided which is composed of a ceramic like metal such as iridium oxid or titanium nitrate. The second layer is formed with a rough surface to provide an increased friction factor and retention on a balloon during advancement of the stent delivery system through the vessel.

In DE 199 16 086 a stent is disclosed comprising a surface which is coated with a ceramic material to achieve a rough surface. On the rough surface, a further thin layer can be provided comprising a drug wherein the layer is biologically degraded and whereby the drug is gradually released.

In WO 99/07308 a stent is disclosed wherein a portion of a stent supporting structure is encapsulated with a thin flexible coating made of a polymer which can be used as a carrier for supporting therapeutic agents and drugs. Furthermore, the supporting structure, preferably only the portion which is not encapsulated by the thin flexible coating, is further processed to form a porous exterior surface. Said porous exterior surface renders the exposed portions of the supporting structure, such as the proximal and distal ends more biocompatible by promoting tissue in-growth while reducing the formation of blood clots.

Said stents of the prior art have the disadvantage that they are complicated to manufacture and expensive.

The object of the present invention is therefore to provide an improved stent, which can be manufactured at low costs and which can further avoid thrombus formation and a stenosis.

This object is achieved by the features of the claims.

According to the invention as claimed in claim 1 a stent is provided comprising at least an outer surface portion which is roughened to a predetermined extent and wherein a drug or a therapeutic agent can be applied to said surface.

This has the advantage, that the stent does not have to be provided with an additional drug deposit e.g. a polymer layer suitable to carry a drug or therapeutic agent. Instead the drug can be applied directly to the rough surface and released over a predetermined time after the stent has been placed in a desired location of a lumen. Furthermore a roughened exterior surface decrease in-stent restenosis, since cells can attach more easily to said surface than to a smooth one which results in that endothelial ingrowth is accelerated. Intima cells can grow on the rough surface and attach themselves, wherein the endothelialization of the vessel or lumen is promoted. The endothelial cell layer is very smooth and therefore thrombus formation and a stenosis can be avoided.

In a preferred embodiment the drug e.g. Tacrolimus is applied to the rough surface by spraying. This has the advantage, that the application of the drug is effective, simple and inexpensive.

In a further preferred embodiment of the invention imperfections such as e.g. burrs are removed before at least a portion of the surface of the stent is roughened. This has the advantage that the surface can be roughened more uniform which leads to better flow dynamics. Thus less turbulences can occur on the surface which results in a reduction of restenosis.

According to the invention the surface is roughened to a predetermined extent by sandblasting. Moreover, sandblasting results in an improvement of the fatigue behaviour. Further the durability of the stent and the surface bonding can be improved. The rough surface also provides an increased surface area for an attachment of a drug or therapeutic agent. Further a stent with a thinner wall with higher radial force and therefore less material can be achieved which also leads to a decrease of restenosis. With sandblasting the surface can be better controlled and produced and further a more uniform and trauma less surface can be achieved.

Furthermore, the use of corundum for sandblasting results in a surface which is technically different from a normal sandblasted surface. It has the advantage that less energy has to be used and/or less time for this finishing sandblasting than for a sandblasting process to remove burrs. Further the sandblasted surface has less depth with regard to the "cavities". Furthermore the chemical behaviour of such a stent is different from commonly known electropolished stents. The surface chemistry is different due to the incorporation of sand particles into the surface. An immediately repassivated surface leads to more chemically stable passive layers than surfaces which have been passivated in equilibrium.

When blasting the surface the resulting lattice imperfections (e.g. vacancy, dislocation) and further possible phase transitions lead to an increased surface energy and thus to a surface which is chemically more reactive. This can lead to a faster chemical running and/or to additional chemical reactions than in the equilibrium.

In a further preferred embodiment the stent is annealed after the surface has been roughened to a predetermined extent to make him more flexible.

The invention will now be described with reference to the figures, in which
figures 1 to 7 show sandblasted exterior and side surfaces of a stent in different resolutions,
figures 8 to 14 show sandblasted interior and side surfaces of a stent in different resolutions,
figure 15 shows a table 1, including a list of samples of stents which are used for studying content and release of a drug applied on their surface,
figure 16 show tables 2 and 3 wherein the results of several samples regarding their content are listed,
figure 17 show tables 4 and 5, wherein samples are studied regarding release of the drug Tacrolimus, and
figure 18 shows a diagram, wherein the release of Tacrolimus of samples with respect to time is shown.

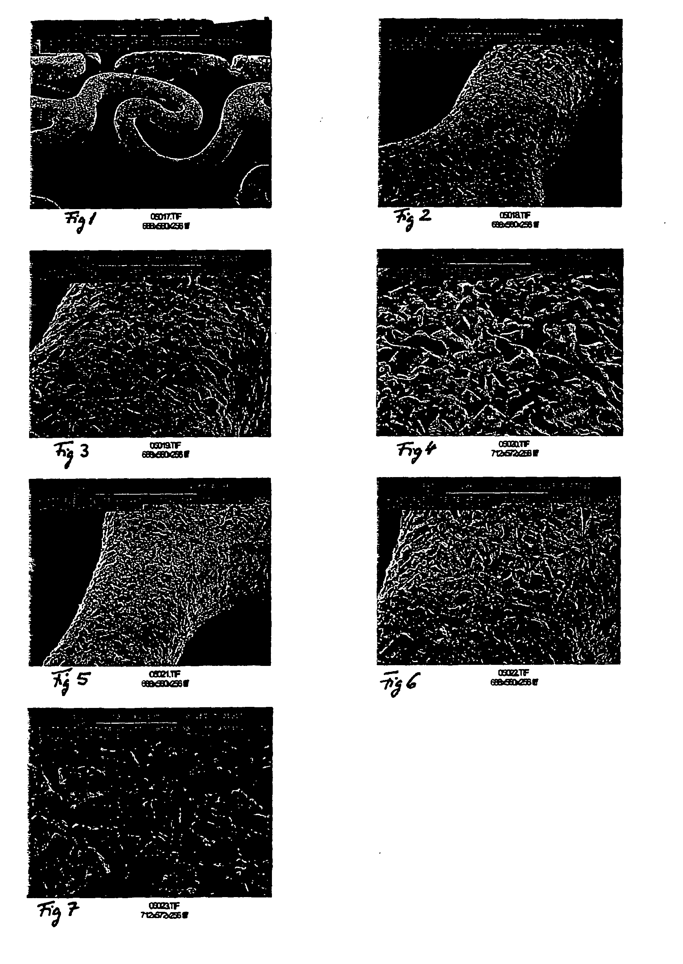

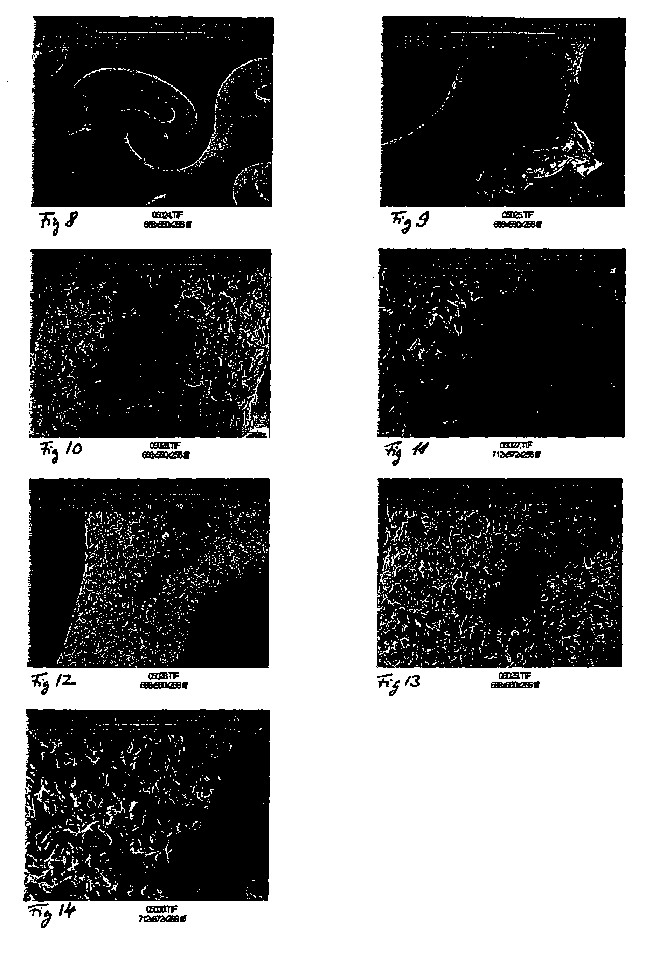

In an embodiment of a stent according to the invention, as shown in figures 1 to 14, the complete surface, i.e. exterior surface, interior surface and side surfaces, is sandblasted by using corundum. It is obvious that the invention is not limited to said embodiment and that also only portions of the surface can be roughened as claimed.

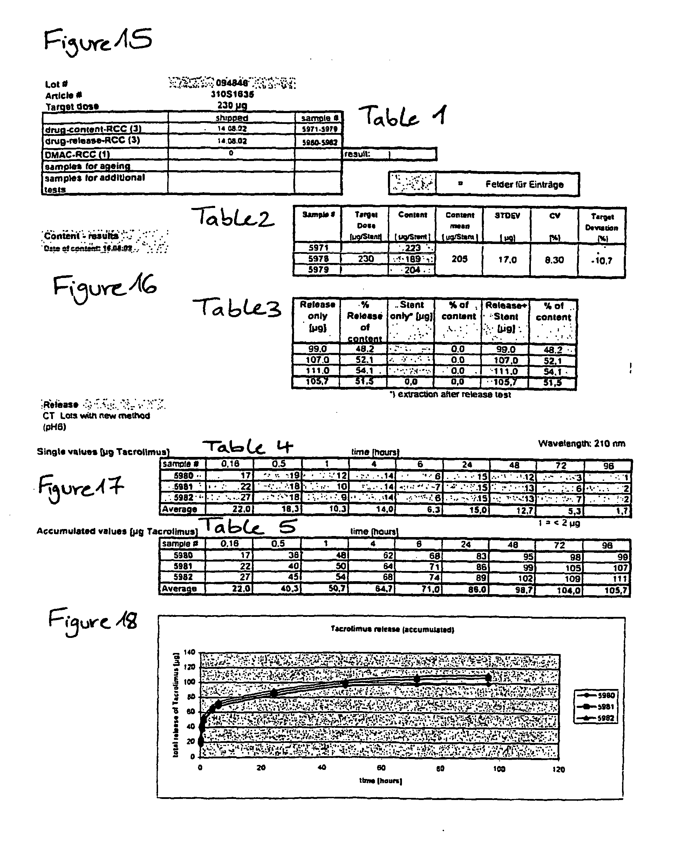

In figures 15 to 18 content and release of samples of stents are studied. In this connection normal manufactured stents are compared with stents which are further processed according to the invention. Based on the examples shown in the figures the improved properties of stents according to the invention can be demonstrated.

## Claims

1. A vascular stent for placement in a body lumen, said stent being expandable from a contracted state to an expanded state and comprising :
a) an exterior surface, an interior surface and side surfaces,
b) wherein at least a portion of the exterior surface and the interior surface is roughened to a predetermined extent for coating with a drug, wherein the surfaces are roughened by sandblasting.

2. A stent according to claim 1, comprising at least a portion of the side surfaces which is roughened to a predetermined extent.

3. A stent according to claim 1 or 2, wherein the surface is roughened by sandblasting with sand, glass beads or corundum.

4. A stent according to any of claims 1 to 3, wherein the outer surface and/or the inner surface and/or side surfaces are coated with a drug, preferably Tacrolimus.

5. A stent according to any of claims 1 to 4, wherein said stent comprises a stainless steel.

6. A balloon catheter device for inserting a tubular stent, comprising a stent as defined in any of claims 1 to 5.

7. A method for fabricating a vascular stent for placement in a body lumen, said method comprising the following steps:
a) forming a tube which can be employed from a contracted state to an expanded state, said stent having an exterior surface, an interior surface and side surfaces,
b) roughening of at least a portion of the exterior surface and the interior surface to a predetermined extent, wherein the surfaces are roughened by sandblasting,
c) coating of said surface with a drug.

8. A method according to claim 7, wherein at least a portion of the side surfaces is roughened to a predetermined extent.

9. A method according to any of claims 7 and 8 including an additional step d) after step a) wherein imperfections such as sharp edges and burrs are removed from the surface of the tube.

10. A method according to claim 9, wherein the imperfections are removed by burring.

11. A method according to claim 9 or 10, wherein the imperfections are removed by electropolishing.

12. A method according to any of claims 9 to 11, wherein the imperfections are removed by sandblasting.

13. A method according to any of claims 7 to 12, wherein sand is used for sandblasting.

14. A method according to any of claims 7 to 13, wherein glass beads are used for sandblasting.

15. A method according to any of claims 7 to 14, wherein corundum is used for sandblasting.

16. A method according to any of claims 7 to 15, wherein the drug is Tacrolimus.

17. A method according to any of claims 7 to 16, wherein the drug is applied by spraying on the roughened surface.

18. A method according to any of claims 7 to 17, wherein the tube is annealed.

## Patentansprüche

1. Vascularstent zur Platzierung in einem Körperlumen, wobei der Stent von einem kontrahierten Zustand in einen expandierten Zustand expandierbar ist und
a) eine äußere Oberfläche, eine innere Oberfläche und Seitenoberflächen, umfasst, wobei
b) wenigstens ein Teil der äußeren Oberfläche und der inneren Oberfläche in einem vorbestimmten Umfang zur Beschichtung mit einem Arzneimittel geraut ist, wobei die Oberflächen durch Sandstrahlen geraut sind.

2. Stent gemäß Anspruch 1, bei dem wenigstens ein Teil der Seitenoberflächen in einem vorbestimmten Umfang geraut ist

3. Stent gemäß Anspruch 1 oder 2, wobei die Oberfläche durch Sandstrahlen mit Sand, Glasbeads oder Korund geraut ist.

4. Stent gemäß irgendeinem der Ansprüche 1 bis 3, wobei die äußere Oberfläche und/oder die innere Oberfläche und/oder die Seitenoberflächen mit einem Arzneimittel, bevorzugt Tacrolimus, beschichtet sind.

5. Stent gemäß irgendeinem der Ansprüche 1 bis 4, wobei der Stent einen nichtrostenden Stahl umfasst.

6. Ballonkatheter-Vorrichtung zum Einsetzen eines tubularen Stents, welcher einen Stent gemäß irgendeinem der Ansprüche 1 bis 5 umfasst.

7. Verfahren zur Herstellung eines Vaskularstents zur Platzierung in einem Körperlumen, wobei das Verfahren folgende Schritte umfasst:
a) Bildung einer Röhre, welche von einem kontrahierten Zustand in einen expandierten Zustand gebracht werden kann, wobei der Stent eine äußere Oberfläche, eine innere Oberfläche und Seitenoberflächen aufweist,
b) Rauen wenigstens eines Teils der äußeren Oberfläche und der inneren Oberfläche bis zu einem vorbestimmten Umfang, wobei die Oberflächen durch Sandstrahlen geraut werden,
c) Beschichten der Oberfläche mit einem Arzneimittel.

8. Verfahren gemäß Anspruch 7, wobei wenigstens ein Teil der Seitenoberflächen bis zu einem vorbestimmten Umfang geraut wird.

9. Verfahren gemäß irgendeinem der Ansprüche 7 und 8, umfassend einen zusätzlichen Schritt d) nach Schritt a), in dem Imperfektionen, wie beispielsweise scharfe Kanten und Grate, von der Oberfläche der Röhre entfernt werden.

10. Verfahren gemäß Anspruch 9, wobei die Imperfektionen durch Entgraten entfernt werden.

11. Verfahren gemäß Anspruch 9 oder 10, wobei die Imperfektionen durch Elektropolieren entfernt werden.

12. Verfahren gemäß irgendeinem der Ansprüche 9 bis 11, wobei die Imperfektionen durch Sandstrahlen entfernt werden.

13. Verfahren gemäß irgendeinem der Ansprüche 7 bis 12, wobei Sand zum Sandstrahlen verwendet wird.

14. Verfahren gemäß irgendeinem der Ansprüche 7 bis 13, wobei Glasbeads zum Sandstrahlen verwendet werden.

15. Verfahren gemäß irgendeinem der Ansprüche 7 bis 14, wobei Korund zum Sandstrahlen verwendet wird.

16. Verfahren gemäß irgendeinem der Ansprüche 7 bis 15, wobei das Arzneimittel Tacrolimus ist.

17. Verfahren gemäß irgendeinem der Ansprüche 7 bis 16, wobei das Arzneimittel durch Aufsprühen auf die geraute Oberfläche aufgetragen wird.

18. Verfahren gemäß irgendeinem der Ansprüche 7 bis 17, wobei die Röhre geglüht/getempert wird.

## Revendications

1. Stent vasculaire pour la mise en place dans une lumière corporelle, ledit stent étant expansible a partir d'un état contracté jusqu'a un état dilaté et comprenant :
a) une surface extérieure, une surface intérieure et des surfaces latérales,
b) dans lequel au moins une partie de la surface extérieure et de la surface intérieure est rendue rugueuse jusqu'à une étendue prédéterminée, pour être revêtue d'un médicament, les surfaces étant rendues rugueuses par une opération de sablage.

2. Stent selon la revendication 1, comprenant au moins une portion des surfaces latérales qui est rendue rugueuse jusqu'à une étendue prédéterminée.

3. Stent selon la revendication 1 ou la revendication 2, dans lequel la surface est rendue rugueuse par une opération de sablage, avec du sable, des billes de verre ou du corindon.

4. Stent selon l'une quelconque des revendications 1 à 3, dans lequel la surface extérieure et/ou la surface intérieure et/ou les surfaces latérales sont revêtues d'un médicament, de préférence du Tacrolimus.

5. Stent selon l'une quelconque des revendications 1 a 4, dans lequel ledit stent comprend un acier inoxydable.

6. Dispositif à cathéter à ballonnet pour l'insertion d'un stent tubulaire, comprenant un stent tel que défini dans l'une quelconque des revendications 1 à 5.

7. Procédé pour la fabrication d'un stent vasculaire pour la mise en place dans une lumière corporelle, ledit procédé comprenant les étapes suivantes :
a) formation d'un tube qui peut être employé depuis un état contracté jusqu'à un état dilaté, ledit stent ayant une surface extérieure, une surface intérieure et des surfaces latérales,
b) opération consistant à rendre rugueuse au moins une partie de la surface extérieure et de la surface intérieure jusqu'à une étendue prédéterminée, les surfaces étant rendues rugueuses par sablage,
c) revêtement de ladite surface d'un médicament.

8. Procédé selon la revendication 7, dans lequel au moins une partie des surfaces latérales est rendue rugueuse jusqu'à une étendue prédéterminée.

9. Procédé selon l'une quelconque des revendications 7 et 8, comprenant une étape supplémentaire d) après l'étape a) dans laquelle des imperfections, telles que des bords tranchants et des bavures, sont enlevées de la surface du tube.

10. Procédé selon la revendication 9, dans lequel les imperfections sont enlevées par ébavurage.

11. Procédé selon la revendication 9 ou la revendication 10, dans lequel les imperfections sont enlevées par électropolissage.

12. Procédé selon l'une quelconque des revendications 9 à 11, dans lequel les imperfections sont enlevées par sablage.

13. Procédé selon l'une quelconque des revendications 7 à 12, dans lequel le sable est utilisé pour l'opération de sablage.

14. Procédé selon l'une quelconque des revendications 7 à 13, dans lequel on utilise des billes de verre pour le sablage.

15. Procédé selon l'une quelconque des revendications 7 à 14, dans lequel on utilise du corindon pour le sablage.

16. Procédé selon l'une quelconque des revendications 7 à 15, dans lequel le médicament est le Tacrolimus.

17. Procédé selon l'une quelconque des revendications 7 à 16, dans lequel le médicament est appliqué par pulvérisation sur la surface rendue rugueuse.

18. Procédé selon l'une quelconque des revendications 7 à 17, dans lequel le tube est recuit.
